# EUROPÄISCHE PATENTANMELDUNG

(11) **EP 3 202 394 A1**
(43) Veröffentlichungstag der Anmeldung: **09.08.2017**
(21) Anmeldenummer: 16190103.8
(22) Anmeldetag: 02.07.2010
(51) Int. Cl.: A61K 9/10, A61K 47/10, C07K 14/62, C12N 15/81, A61K 47/18

(54) **WÄSSRIGE INSULINZUBEREITUNGEN ENTHALTEND METHIONIN**

(30) Priorität: 06.07.2009 DE 102009031748; 27.03.2010 DE 102010013134
(62) Teilanmeldung aus: 10730443.8
(71) Anmelder: Sanofi-Aventis Deutschland GmbH, 65929 Frankfurt am Main (DE)
(72) Erfinder: SCHOETTLE, Isabell, 65926 Frankfurt am Main (DE); HAGENDORF, Annika, 65926 Frankfurt am Main (DE); FUERST, Christiane, 65926 Frankfurt am Main (DE); HAUCK, Gerrit, 65926 Frankfurt am Main (DE); SIEFKE-HENZLER, Verena, 65926 Frankfurt am Main (DE); KAMM, Walter, 65926 Frankfurt am Main (DE); SCHNIEDERS, Julia, 65926 Frankfurt am Main (DE)
(74) Vertreter: Weickmann & Weickmann PartmbB

(57) **Zusammenfassung**

Die Erfindung betrifft eine wässrige pharmazeutische Formulierung mit einem Insulin, einem Insulinanalogon oder einem Insulinderivat und Methionin; sowie deren Herstellung, Verwendung zur Behandlung von Diabetes mellitus und ein Arzneimittel zur Behandlung von Diabetes Mellitus.

## Beschreibung

Die Erfindung betrifft eine wässrige pharmazeutische Formulierung mit einem Insulin, einem Insulinanalogon oder einem Insulinderivat und Methionin; sowie deren Herstellung, Verwendung zur Behandlung von Diabetes mellitus und ein Arzneimittel zur Behandlung von Diabetes Mellitus.

Weltweit leidet eine zunehmende Zahl an Menschen an Diabetes mellitus. Darunter sind viele sogenannte Typ I-Diabetiker, für die die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen. Beim Gesunden ist die Insulinfreisetzung durch den Pankreas strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subcutane Applikation von Insulin erreicht in der Regel die oben beschriebene Qualität der physiologischen Regulation der Blutglucose nicht annähernd. Häufig kommt es zu Entgleisungen der Blutglucose nach oben oder unten, die in ihren schwersten Formen lebensbedrohlich sein können. Daneben stellen jedoch auch über Jahre erhöhte Blutglucosespiegel ohne anfängliche Symptome ein erhebliches Gesundheitsrisiko dar. Die großangelegte DCCT-Studie in den USA (The Diabetes Control and Complications Trial Research Group (1993) N. Engl. J. Med. 329, 977-986) wies eindeutig nach, daß chronisch erhöhte Blutglucosespiegel wesentlich für die Entwicklung diabetischer Spätschäden verantwortlich sind. Diabetische Spätschäden sind mikro- und makrovaskuläre Schädigungen, die sich u.U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, daß eine verbesserte Therapie des Diabetes in erster Linie darauf abzielen muß, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäuren und/oder Addition/Entfernen wenigstens eines Aminosäurerestes von dem entsprechenden, ansonsten gleichen natürlich vorkommenden Insulin unterscheiden. Es kann sich dabei auch um Aminosäuren handeln, die nicht natürlich vorkommen.

Insulinderivate sind Derivate von natürlich vorkommendem Insulin oder einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen. In der Regel haben Insulinderivate und Insulinanaloga gegenüber humanem Insulin eine etwas veränderte Wirkung.

Insulinanaloga mit beschleunigtem Wirkungseintritt werden in EP 0 214 826, EP 0 375 437 und EP 0 678 522 beschrieben. EP 0 124 826 bezieht sich u.a. auf Substitutionen von B27 und B28. EP 0 678 522 beschreibt Insulinanaloga, die in der Position B29 verschiedene Aminosäuren, vorzugsweise Prolin, aufweisen, jedoch nicht Glutaminsäure.
EP 0 375 437 umfaßt Insulinanaloga mit Lysin oder Arginin in B28, die optional zusätzlich in B3 und/oder A21 modifiziert sein können.

In der EP 0 419 504 werden Insulinanaloga offenbart, die gegen chemische Modifikationen geschützt sind, in dem Asparagin in B3 und wenigstens eine weitere Aminosäure in den Positionen A5, A15, A18 oder A21 verändert sind.

In der Regel haben Insulinderivate und Insulinanaloga gegenüber humanem Insulin eine etwas veränderte Wirkung.

In der WO 92/00321 werden Insulinanaloga beschrieben, bei denen wenigstens eine Aminosäure der Positionen B1-B6 durch Lysin oder Arginin ersetzt ist. Derartige Insuline weisen gemäß WO 92/00321 eine verlängerte Wirkung auf. Eine verzögerte Wirkung weisen auch die in der EP-A 0 368 187 beschriebenen Insulinanaloga auf. Das Konzept der intensivierten Insulintherapie versucht das Gesundheitsrisiko abzumindern, indem eine stabile Kontrolle des Blutzuckerspiegels durch frühe Gabe von Basalinsulinen angestrebt wird. Ein Beispiel für ein gängiges Basalinsulin ist das Medikament Lantus^{®} (Wirkstoff: Insulin Glargin = Gly (A21), Arg (B31), Arg (B32) Humaninsulin). Generell gilt es, bei der Entwicklung neuer, verbesserter Basalinsuline die Zahl hypoglykämischer Ereignisse zu minimieren. Ein ideales Basalinsulin wirkt dabei sicher in jedem Patienten mindestens 24 Stunden. Idealerweise setzt die Insulinwirkung verzögert und mit einem möglichst flachen Zeit- / Wirkungsprofil ein, so dass die Gefahr einer kurzfristigen Unterzuckerung deutlich minimiert ist und die Applikation sogar ohne vorherige Einnahme von Nahrungsmitteln erfolgen kann. Eine gute Versorgung mit Basalinsulin ist dann gegeben, wenn die Insulinwirkung möglichst lange gleichbleibend anhält, d.h. der Körper mit einer konstanten Menge Insulin versorgt wird. Damit ist die Gefahr hypoglykämischer Ereignisse gering und eine patienten- und tagesspezifische Variabilität minimiert. Das pharmakokinetische Profil eines idealen Basalinsulins sollte also durch einen verzögerten Wirkeintritt und durch eine verzögerte, d.h. lang anhaltende und gleichmäßige Wirkung gekennzeichnet sein.

Die auf dem Markt befindlichen Insulinzubereitungen von natürlich vorkommenden Insulinen zur Insulinsubstitution unterscheiden sich in der Herkunft des Insulins (z.B. Rind, Schwein, Humaninsulin), sowie der Zusammensetzung, womit das Wirkprofil (Wirkeintritt und Wirkdauer) beeinflusst werden kann. Durch Kombination verschiedener Insulinpräparate lassen sich unterschiedlichste Wirkprofile erzielen und möglichst physiologische Blutzuckerwerte einstellen. Die rekombinante DNA Technologie ermöglicht heutzutage die Herstellung von solchen modifizierten Insulinen. Hierzu zählt Insulin Glargin (Gly(A21)-Arg(B31)-Arg(B32)-Humaninsulin) mit einer verlängerten Wirkdauer. Insulin Glargin wird als saure, klare Lösung injiziert und fällt aufgrund seiner Lösungseigenschaften im physiologischen pH-Bereich des Subkutangewebes als stabiles Hexamerassoziat aus. Insulin Glargin wird einmal täglich injiziert und zeichnet sich gegenüber anderen langwirksamen Insulinen durch sein flaches Serumprofil und die damit verbundene Reduktion der Gefahr nächtlicher Hypoglykämien aus (Schubert-Zsilavecz et al., 2:125-130(2001)). Die spezifische Zubereitung des Insulin Glargins, die zur verlängerten Wirkdauer führt, ist im Gegensatz zu bisher beschriebenen Zubereitungen durch einen klare Lösung mit saurem pH-Wert gekennzeichnet. Gerade bei saurem pH-Wert zeigen Insuline jedoch eine verringerte Stabilität und eine erhöhte Aggregationsneigung bei thermischer und physikalisch-mechanischer Belastung, die sich in Form von Trübungen und Ausfällungen (Partikelbildungen) bemerkbar machen kann (Brange et al., J. Ph.Sci 86:517-525(1997)).

Es wurde gefunden, dass solche Insulinanaloga zu dem beschriebenen wünschenswerten basalen Zeit- / Wirkungsprofil führen, wenn die Insulinanaloga durch die Merkmale charakterisiert sind, dass
- das B- Kettenende aus einem amidierten basischen Aminosäurerest wie Lysin bzw. Argininamid besteht, d.h. bei dem amidierten basischen Aminosäurerest am B-Kettenende liegt die Carboxylgruppe der endständigen Aminosäure in ihrer amidierten Form vor, und
- der N-terminale Aminosäurerest der Insulin A-Kette ein Lysin- oder Argininrest ist, und
- die Aminosäureposition A8 durch einen Histidinrest besetzt wird, und
- die Aminosäureposition A21 durch einen Glycinrest besetzt wird, und
- zwei Substitutionen neutraler Aminosäuren durch saure Aminosäuren, zwei Additionen negativ geladener Aminosäurereste oder je eine solche Substitution und eine solche Addition jeweils in den Positionen A5, A15, A18, B-1, B0, B1, B2, B3 und B4 erfolgt sind.

Allen wässrigen Formulierungen von Insulinen, Insulinanaloga und Insulinderivaten ist gemein, dass die genannten Proteine chemisch nicht völlig stabil sind, sondern dass sich in Abhängigkeit von der Zeit, der Lagertemperatur, der Bewegung, der die Formulierung unterworfen ist uvm. eine Reihe von molekularen Prozessen bei den Insulinen, Insulinanaloga und Insulinderivaten ablaufen können, die nachteilig für die Qualität der Formulierung sind. Ein Stoff, welcher die chemische Stabilität der Insuline, Insulinanaloga und Insulinderivate beeinträchtigt, ist Sauerstoff, dessen Kontakt mit den entsprechenden Formulierungen aufgrund seines Vorhandenseins in der Luft-insbesondere bei Formulierungen in Verpackungen für die Mehrfachgaben - nicht zu verhindern ist. Man nimmt an, dass es u.a. das oxidative Potential von Sauerstoff ist, welche die Beeinträchtigungen der chemischen Stabilität hervorruft.

Es wurde nun überraschenderweise gefunden, dass die Beimengung der Aminosäure Methionin zu Formulierungen von Insulinen, Insulinanaloga und Insulinderivaten zu einer verbesserten Stabilität dieser Proteine führt.

Ein Gegenstand der Erfindung ist daher eine wässrige pharmazeutische Formulierung enthaltend ein Insulin, ein Insulinanalogon oder ein Insulinderivat, oder ein pharmakologisch tolerierbares Salz davon, und Methionin.

Ein weiterer Gegenstand der Erfindung ist eine pharmazeutische Formulierung wie oben beschrieben, wobei das Insulin ausgewählt wird aus einer Gruppe enthaltend menschliches Insulin, Schweine-Insulin und Insulin des Rindes.

Ein weiterer Gegenstand der Erfindung ist eine pharmazeutische Formulierung wie oben beschrieben, wobei das Insulinanalogon ausgewählt wird aus einer Gruppe enthaltend Gly(A21), Arg(B31), Arg(B32)-Humaninsulin, Lys(B3), Glu(B29)-Humaninsulin, Asp(B28)-Humaninsulin, Lys(B28) Pro(B29)-Humaninsulin, Des(B30)-Humaninsulin und ein Insulinanalogon der Formel I 
S ——————— S
1 5 | 10| 15 20
A0 G I V E A5 C C H S I C S L Y A15 L E A18 Y C G
| (SEQ ID NO: 1) A-Kette
S S
|
S S
| |
B-1 B0 B1 B2 B3 B4 H L C G S H L V E A L Y L V C G E R G F F Y
1 5 10 15 20 25

T P B29 B30 B31 B32 (SEQ ID NO: 2)
B-Kette
30
 wobei
- A0: Lys oder Arg;
- A5: Asp, Gln oder Glu;
- A15: Asp, Glu oder Gln;
- A18: Asp, Glu oder Asn;
- B-1: Asp, Glu oder eine Aminogruppe;
- B0: Asp, Glu oder eine chemische Bindung;
- B1: Asp, Glu oder Phe;
- B2: Asp, Glu oder Val,
- B3: Asp, Glu oder Asn;
- B4: Asp, Glu oder Gln;
- B29: Lys oder einer chemischen Bindung;
- B30: Thr oder einer chemischen Bindung;
- B31: Arg, Lys oder einer chemischen Bindung;
- B32: Arg-Amid, Lys-Amid oder einer Aminogruppe
entspricht, wobei zwei Aminosäurereste der Gruppe enthaltend A5, A15, A18, B-1, B0, B1, B2, B3 und B4 gleichzeitig und unabhängig voneinander Asp oder Glu entsprechen, insbesondere bei der das Insulinanalogon ausgewählt ist aus einer Gruppe enthaltend:
Arg (A0), His (A8), Glu (A5), Asp (A18), Gly (A21), Arg (B31), Arg (B32) - NH₂ Humaninsulin, Arg (A0), His (A8), Glu (A5), Asp (A18), Gly (A21), Arg (B31), Lys (B32) - NH₂ Humaninsulin, Arg (A0), His (A8), Glu (A15), Asp (A18), Gly (A21), Arg (B31), Arg (B32)- NH₂ Humaninsulin, Arg (A0), His (A8), Glu (A15), Asp (A18), Gly (A21), Arg (B31), Lys (B32) - NH₂ Humaninsulin, Arg (A0), His (A8), Glu(A5), Glu (A15), Gly (A21), Arg (B31), Arg (B32)- NH₂ Humaninsulin, Arg (A0), His (A8), Glu (A5), Glu (A15), Gly (A21), Arg (B31), Lys (B32) - NH₂ Humaninsulin, Arg (A0), His(A8), Glu (A5), Gly (A21), Asp (B3), Arg (B31), Arg (B32) - NH₂ Humaninsulin, Arg (A0), His(A8), Glu (A5), Gly (A21), Asp (B3), Arg (B31), Lys (B32) - NH₂ Humaninsulin, Arg (A0), His (A8), Glu (A15), Gly (A21), Asp (B3), Arg (B31), Arg (B32) - NH₂ Humaninsulin, Arg (A0), His (A8), Glu (A15), Gly (A21), Asp (B3), Arg (B31), Lys (B32) - NH₂ Humaninsulin, Arg (A0), His (A8), Asp (A18), Gly (A21), Asp (B3), Arg (B31), Arg (B32)- NH₂ Humaninsulin, Arg (A0), His (A8), Asp (A18), Gly (A21), Asp (B3), Arg (B31), Lys (B32)- NH₂ Humaninsulin, Arg (A0), His(A8), Gly (A21), Asp (B3), Glu (B4), Arg (B31), Arg (B32)- NH₂ Humaninsulin, Arg (A0), His (A8), Gly (A21), Asp (B3), Glu (B4), Arg (B31), Lys (B32)- NH₂ Humaninsulin, Arg (A0), His (A8), Glu (A5), Gly (A21), Glu (B4), Arg (B31), Arg (B32)- NH₂ Humaninsulin, Arg (A0), His (A8), Glu (A5), Gly (A21), Glu (B4), Arg (B31), Lys (B32) - NH₂ Humaninsulin, Arg (A0), His (A8), Glu (A15), Gly (A21), Glu (B4), Arg (B31), Arg (B32) - NH₂ Humaninsulin, Arg (A0), His (A8), Glu (A15), Gly (A21), Glu (B4), Arg (B31), Lys (B32) - NH₂ Humaninsulin, Arg (A0), His (A8), Asp (A18), Gly (A21), Glu (B4), Arg (B31), Arg (B32) - NH₂ Humaninsulin, Arg (A0), His (A8), Asp (A18), Gly (A21), Glu (B4), Arg (B31), Lys (B32) - NH₂ Humaninsulin, Arg (A0), His (A8), Glu (A5), Gly (A21), Glu (B0), Arg (B31), Arg (B32)- NH₂ Humaninsulin, Arg (A0), His (A8), Glu (A5), Gly (A21), Glu (B0), Arg (B31), Lys (B32)- NH₂ Humaninsulin, Arg (A0), His (A8), Glu (A15), Gly (A21), Glu (B0), Arg (B31), Arg (B32) - NH₂ Humaninsulin, Arg (A0), His (A8), Glu (A15), Gly (A21), Glu (B0), Arg (B31), Lys (B32)- NH₂ Humaninsulin, Arg (A0), His (A8), Asp (A18), Gly (A21), Glu (B0), Arg (B31), Arg (B32)- NH₂ Humaninsulin, Arg (A0), His (A8), Asp (A18),Gly (A21), Glu (B0), Arg (B31), Lys (B32) - NH₂ Humaninsulin, Arg (A0), His (A8), Glu (A5), Gly (A21), Asp (B1), Arg (B31), Arg (B32)- NH₂ Humaninsulin, Arg (A0), His (A8), Glu (A5), Gly (A21), Asp (B1), Arg (B31), Lys (B32)- NH₂ Humaninsulin, Arg (A0), His (A8), Glu (A15), Gly (A21), Asp (B1), Arg (B31), Arg(B32) - NH₂ Humaninsulin, Arg (A0), His (A8), Glu (A15), Gly (A21), Asp (B1), Arg (B31), Lys (B32) - NH₂ Humaninsulin, Arg (A0), His (A8), Asp (A18), Gly (A21), Asp (B1), Arg (B31), Arg (B32)- NH₂ Humaninsulin, Arg (A0), His (A8), Asp (A18), Gly (A21), Asp (B1), Arg (B31), Lys (B32) - NH₂ Humaninsulin, Arg (A0), His (A8), Gly (A21), Glu (B0), Asp (B1), Arg (B31), Arg (B32) - NH₂ Humaninsulin, Arg (A0), His (A8), Gly (A21), Glu (B0), Asp (B1), Arg (B31), Lys (B32) - NH₂ Humaninsulin, Arg (A0), His (A8), Asp (A18), Gly (A21), Asp (B3), Arg (B30), Arg (B31) - NH₂ Humaninsulin, Arg (A0), His (A8), Asp (A18), Gly (A21), Asp (B3), Arg (B30), Lys (B31) - NH₂ Humaninsulin.

Ein weiterer Gegenstand der Erfindung ist eine pharmazeutische Formulierung wie oben beschrieben, wobei das Insulinanalogon ausgewählt wird aus einer Gruppe enthaltend ein Insulinanalogon der Formel II 
S S
1 5 | 10 |
A-1 A0 A1 I V E A5 C C H S I C S L Y A15 L E A18 Y C A21
A-Kette
(SEQ ID NO: 3)
S S
S S
B-1 B0 B1 V B3 B4 H L C G S H L V E A L Y L V C G E R G F F Y
1 5 10 15 20 25
T P B29 B30 B31 B32 (SEQ ID NO: 4) B-Kette
30
 wobei
A-1 Lys, Arg oder einer Aminogruppe;
A0 Lys, Arg oder einer chemischen Bindung;
A1 Arg oder Gly;
A5 Asp, Glu oder Gln;
A15 Asp, Glu oder Gln;
A18 Asp, Glu oder Asn;
A21 Ala, Ser, Thr oder Gly;
B-1 Asp, Glu oder eine Aminogruppe;
B0 Asp, Glu oder eine chemische Bindung;
B1 Asp, Glu, Phe oder eine chemische Bindung;
B3 Asp, Glu oder Asn;
B4 Asp, Glu oder Gln;
B29 Arg, Lys oder einer Aminosäure ausgewählt aus einer Gruppe enthaltend die Aminosäuren Phe, Ala, Thr, Ser, Val, Leu, Glu oder Asp, oder einer chemischen Bindung;
B30 Thr oder einer chemischen Bindung;
B31 Arg, Lys oder einer chemischen Bindung;
B32 Arg-Amid oder Lys-Amid
entspricht, wobei nicht mehr als ein Aminosäurerest der Gruppe enthaltend A5, A15, A18, B-1, B0, B1, B2, B3 und B4 gleichzeitig und unabhängig voneinander Asp oder Glu entsprechen, insbesondere bei der das Insulinanalogon ausgewählt ist aus einer Gruppe enthaltend:
Arg (A-1), Arg (A0), Glu (A5), His (A8), Gly (A21), Arg (B30) - NH₂ Humaninsulin, Arg (A-1), Arg (A0), Glu (A5), His (A8), Gly (A21), Lys (B30) - NH₂ Humaninsulin, Arg (A-1), Arg (A0), Glu (A15), His (A8), Gly (A21), Arg (B30) - NH₂ Humaninsulin, Arg (A-1), Arg (A0), Glu (A15), His (A8), Gly (A21), Lys (B30) - NH₂ Humaninsulin, Arg (A-1), Arg (A0), Asp (A18), His (A8), Gly (A21), Arg (B30) - NH₂ Humaninsulin, Arg (A-1), Arg (A0), Asp (A18), His (A8), Gly (A21), Arg (B30) - NH₂ Humaninsulin, Arg (A-1), Arg (A0), His (A8), Gly (A21), Glu (B0), Arg (B30) - NH₂ Humaninsulin, Arg (A-1), Arg (A0), His (A8), Gly (A21), Glu (B0), Lys (B30) - NH₂ Humaninsulin, Arg (A-1), Arg (A0), His (A8), Gly (A21), Asp (B3), Arg (B30) - NH₂ Humaninsulin, Arg (A-1), Arg (A0), His (A8), Gly (A21), Asp (B3), Lys (B30) - NH₂ Humaninsulin, Arg (A-1), Arg (A0), His (A8), Gly (A21), Glu (B4), Arg (B30) - NH₂ Humaninsulin, Arg (A-1), Arg (A0), His (A8), Gly (A21), Glu (B4), Lys (B30) - NH₂ Humaninsulin, Arg (A0), His (A8), Gly (A21), Arg (B31), Arg (B32) - NH₂ - Humaninsulin, Arg (A0), His (A8), Gly (A21), Arg (B31), Lys (B32) - NH₂ - Humaninsulin, Arg (A0), Glu (A5), His (A8), Gly (A21), Arg (B31), Arg (B32) - NH₂ - Humaninsulin, Arg (A0), Glu (A5), His (A8), Gly (A21), Arg (B31), Lys (B32) - NH₂ - Humaninsulin, Arg (A0), Asp (A18), His (A8), Gly (A21), Arg (B31), Arg (B32) - NH₂ - Humaninsulin, Arg (A0), Asp (A18), His (A8), Gly (A21), Arg (B31), Lys (B32) - NH₂ - Humaninsulin, Arg (A0), Glu (A15), His (A8), Gly (A21), Arg (B31), Arg (B32) - NH₂ - Humaninsulin, Arg (A0), Glu (A15), His (A8), Gly (A21), Arg (B31), Lys (B32) - NH₂ - Humaninsulin, Arg (A0), His (A8), Gly (A21), Asp (B3), Arg (B31), Arg (B32) - NH₂ - Humaninsulin, Arg (A0), His (A8), Gly (A21), Asp (B3), Arg (B31), Lys (B32) - NH₂ - Humaninsulin, Arg (A0), His (A8), Gly (A21), Glu (B4), Arg (B31), Arg (B32) - NH₂ - Humaninsulin, Arg (A0), His (A8), Gly (A21), Glu (B4), Arg (B31), Lys (B32) - NH₂ - Humaninsulin, Arg (A0), His (A8), Gly (A21), Glu (B0), Arg (B31), Arg (B32) - NH₂ - Humaninsulin, Arg (A0), His (A8), Gly (A21), Glu (B0), Arg (B31), Lys (B32) - NH₂ - Humaninsulin, Arg (A0), His (A8), Gly (A21), Arg (B30) - NH₂ - Humaninsulin, Arg (A0), His (A8), Gly (A21), Lys (B30) - NH₂ - Humaninsulin, Arg (A-1), Arg (A0), His (A8), Gly (A21), Arg (B30) - NH₂ - Humaninsulin, Arg (A-1), Arg (A0), His (A8), Gly (A21), Lys (B30) - NH₂ - Humaninsulin, Arg (A0), Arg (A1), His (A8), Gly (A21), Arg (B30) - NH₂ - Humaninsulin, Arg (A0), Arg (A1), His (A8), Gly (A21), Lys (B30) - NH₂ - Humaninsulin, His (A8), Gly (A21), Arg (B31), Arg (B32) - NH₂ - Humaninsulin.

Ein weiterer Gegenstand der Erfindung ist eine pharmazeutische Formulierung wie oben beschrieben, wobei das Insulinderivat ausgewählt wird aus einer Gruppe enthaltend B29-N-myristoyl-des(B30) Humaninsulin, B29-N-palmitoyl-des(B30) Humaninsulin, B29-N-myristoyl Humaninsulin, B29-N-palmitoyl Humaninsulin, B28-N-myristoyl Lys^{B28}Pro^{B29} Humaninsulin, B28-N-palmitoyl-Lys^{B28}Pro^{B29} Humaninsulin, B30-N-myristoyl-Thr^{B29}Lys^{B30} Humaninsulin, B30-N-palmitoyl- Thr^{B29}Lys^{B30} Humaninsulin, B29-N-(N-palmitoyl-γ-glutamyl)-des(B39) Humaninsulin, B29-N-(N-lithocholyl-γ-glutamyl)-des(B30) Humaninsulin, B29-N-(ω-carboxyheptadecanoyl)-des(B30) Humaninsulin and B29-N-(ω-carboxyheptadecanoyl) Humaninsulin.

Ein weiterer Gegenstand der Erfindung ist eine pharmazeutische Formulierung wie oben beschrieben, die
0,001 bis 0,2 mg/ml Zink,
0,1 bis 5,0 mg/ml eines Konservierungsmittels und
5,0 bis 100 mg/ml eines Isotonisierungsmittels enthält und
deren pH-Wert 5 oder kleiner ist.

Ein weiterer Gegenstand der Erfindung ist eine pharmazeutische Formulierung wie oben beschrieben, in dem ein Konservierungsmittel ausgewählt aus einer Gruppe enthaltend Phenol, m-Cresol, Chlorkresol, Benzylalkohol, Parabene vorhanden ist.

Ein weiterer Gegenstand der Erfindung ist eine pharmazeutische Formulierung wie oben beschrieben, in dem ein Isotonisierungsmittel ausgewählt aus einer Gruppe enthaltend Mannitol, Sorbitol, Lactose, Dextrose, Trehalose, Natriumchlorid, Glycerol vorhanden ist.

Ein weiterer Gegenstand der Erfindung ist eine pharmazeutische Formulierung wie oben beschrieben, mit einem pH-Wert im Bereich von pH 2,5 - 4,5, bevorzugt pH 3,0 - 4,0, besonders bevorzugt im Bereich von pH 3,75.

Ein weiterer Gegenstand der Erfindung ist eine pharmazeutische Formulierung wie oben beschrieben, wobei das Insulin, das Insulinanalogon und/oder das Insulinderivat in einer Konzentration von 240 - 3000 nmol/ml vorliegt

Ein weiterer Gegenstand der Erfindung ist eine pharmazeutische Formulierung wie oben beschrieben, bei der Glycerol in einer Konzentration von 20 bis 30 mg/ml vorliegt.

Ein weiterer Gegenstand der Erfindung ist eine pharmazeutische Formulierung wie oben beschrieben, bei der Glycerol in einer Konzentration von 25 mg/ml vorliegt.

Ein weiterer Gegenstand der Erfindung ist eine pharmazeutische Formulierung wie oben beschrieben, bei der m-Cresol in einer Konzentration von 1 bis 3 mg/ml, bevorzugt 2 mg/ml, vorliegt.

Ein weiterer Gegenstand der Erfindung ist eine pharmazeutische Formulierung wie oben beschrieben, bei der Zink in einer Konzentration 0,01 oder 0,03 oder 0,08 mg/ml vorliegt.

Ein weiterer Gegenstand der Erfindung ist eine pharmazeutische Formulierung wie oben beschrieben, bei der noch zusätzlich ein Glucagon-Like Peptide-1 (GLP1) oder ein Analogon oder Derivat davon, oder Exendin-3 bzw. -4 oder ein Analogon oder Derivat davon enthalten ist, bevorzugt Exendin-4 enthalten ist.

Ein weiterer Gegenstand der Erfindung ist eine pharmazeutische Formulierung wie oben beschrieben, bei dem ein Analogon von Exendin-4 ausgewählt wird aus einer Gruppe enthaltend
H-desPro³⁶-Exendin-4-Lys₆-NH₂,
H-des(Pro^{36,37})-Exendin-4-Lys₄-NH₂ und
H-des(Pro^{36,37})-Exendin-4-Lys₅-NH₂,
oder ein pharmakologisch tolerierbares Salz davon, oder bei dem ein Analogon von Exendin-4 ausgewählt wird aus einer Gruppe enthaltend
desPro³⁶ [Asp²⁸]Exendin-4 (1-39),
desPro³⁶ [IsoAsp²⁸]Exendin-4 (1-39),
desPro³⁶ [Met(O)¹⁴, Asp²⁸]Exendin-4 (1-39),
desPro³⁶[Met(O)¹⁴, IsoAsp²⁸]Exendin-4 (1-39),
desPro³⁶ [Trp(O₂)²⁵, Asp²⁸]Exendin-2 (1-39),
desPro³⁶ [Trp(O₂)²⁵, IsoAsp²⁸]Exendin-2 (1-39),
desPro³⁶ [Met(O)¹⁴Trp(O₂)²⁵, Asp²⁸]Exendin-4 (1-39) und
desPro³⁶ [Met(O)¹⁴Trp(O₂)²⁵, IsoAsp²⁸]Exendin-4 (1-39),
oder ein pharmakologisch tolerierbares Salz davon.

Ein weiterer Gegenstand der Erfindung ist eine pharmazeutische Formulierung wie oben beschrieben, bei denen an die C-Termini der Analoga von Exendin-4 das Peptid -Lys₆-NH₂ angefügt ist.

Ein weiterer Gegenstand der Erfindung ist eine pharmazeutische Formulierung wie oben beschrieben, bei dem ein Analogon von Exendin-4 ausgewählt wird aus einer Gruppe enthaltend
H-(Lys)₆- des Pro³⁶ [Asp²⁸]Exendin-4(1-39)-Lys₆-NH₂
des Asp²⁸Pro³⁶, Pro³⁷, Pro₃₈ Exendin-4(1-39) -NH₂,
H-(Lys)₆- des Pro³⁶, Pro³⁷, Pro³⁸ [Asp²⁸]Exendin-4(1-39) -NH₂,
H-Asn-(Glu)₅ des Pro³⁶, Pro³⁷, Pro³⁸ [Asp²⁸]Exendin-4(1-39) -NH₂,
des Pro³⁶, Pro³⁷, Pro³⁸ [Asp²⁸]Exendin-4(1-39)-(Lys)₆-NH₂,
H-(Lys)₆- des Pro³⁶, Pro³⁷, Pro³⁸ [Asp²⁸]Exendin-4(1-39)-(Lys)₆-NH₂,
H-Asn-(Glu)₅- des Pro³⁶, Pro³⁷, Pro³⁸ [Asp²⁸]Exendin-4(1-39)-(Lys)₆-NH₂,
H-(Lys)₆- des Pro³⁶ [Trp(O₂)²⁵, Asp²⁸]Exendin-4(1-39)-Lys₆-NH₂,
H- des Asp²⁸ Pro³⁶, Pro³⁷, Pro³⁸ [Trp(O₂)²⁵]Exendin-4(1-39) -NH₂,
H-(Lys)₆- des Pro³⁶, Pro³⁷, Pro³⁸ [Trp(O₂)²⁵, Asp²⁸]Exendin-4(1-39) -NH₂, H-Asn-(Glu)₅- des Pro³⁶, Pro³⁷, Pro³⁸ [Trp(O₂)²⁵, Asp²⁸]Exendin-4(1-39) -NH₂,
des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶, Pro³⁷, Pro³⁸ [Trp(O₂)²⁵, Asp²⁸]Exendin-4(1-39)-(Lys)₆-NH₂,
H-Asn-(Glu)₅- des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶ [Met(O)¹⁴, Asp²⁸]Exendin-4(1-39)-Lys₆-NH₂,
des Met(O)¹⁴ Asp²⁸ Pro³⁶, Pro³⁷, Pro³⁸ Exendin-4(1-39) -NH₂,
H-(Lys)₆- des Pro³⁶, Pro ³⁷, Pro³⁸ [Met(O)¹⁴, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Asp²⁸]Exendin-4(1-39)-(Lys)₆-NH₂,
H-(Lys)₆- des Pro³⁶, Pro³⁷, Pro³⁸ [Met(O)¹⁴, Asp²⁸]Exendin-4(1-39)-Lys₆-NH₂,
H-Asn-(Glu)₅ des Pro³⁶, Pro³⁷, Pro³⁸ [Met(O)¹⁴, Asp²⁸] Exendin-4(1-39)-(Lys)₆-NH₂,
H-(Lys)₆- des Pro³⁶ [Met(O)¹⁴, Trp(O₂)²⁵, Asp²⁸]Exendin-4(1-39)-Lys₆-NH₂,
des Asp²⁸ Pro³⁶, Pro³⁷, Pro³⁸ [Met(O)¹⁴, Trp(O₂)²⁵]Exendin-4(1-39 -NH₂,
H-(Lys)₆- des Pro³⁶, Pro³⁷, Pro³⁸ [Met(O)¹⁴, Trp(O₂)²⁵, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Trp(O₂)²⁵, Asp²⁸]Exendin-4(1-39)-(Lys)₆-NH₂,
H-(Lys)₆- des Pro³⁶' Pro³⁷, Pro³⁸ [Met(O)¹⁴, Trp(O₂)²⁵, Asp²⁸]Exendin-4(1-39)-(Lys)₆-NH₂,
H-Asn-(Glu)₅- des Pro³⁶, Pro³⁷, Pro³⁸ [Met(O)¹⁴, Trp(O₂)²⁵, Asp²⁸] Exendin-4(1-39)-(Lys)₆-NH₂,
oder ein pharmakologisch tolerierbares Salz davon.

Ein weiterer Gegenstand der Erfindung ist eine pharmazeutische Formulierung wie oben beschrieben, bei dem zusätzlich Arg³⁴, Lys²⁶ (N^{ε}(γ-glutamyl(N^{α}-hexadecanoyl))) GLP-1 (7-37) [liraglutide] oder ein pharmakologisch tolerierbares Salz davon enthalten ist.

Ein weiterer Gegenstand der Erfindung ist eine pharmazeutische Formulierung wie oben beschrieben, bei der Methionin im Konzentrationsbereich von bis zu 10 mg/ml, bevorzugt bis zu 3 mg/ml, vorhanden ist.

Ein weiterer Gegenstand der Erfindung ist ein Verfahren zur Herstellung einer Formulierung wie oben beschrieben, bei dem
(a) die Komponenten in eine wässrige Lösung eingebracht werden und
(b) der pH-Wert eingestellt wird.

Ein weiterer Gegenstand der Erfindung ist eine Verwendung einer Formulierung wie oben beschrieben zur Behandlung von Diabetes mellitus.

Ein weiterer Gegenstand der Erfindung ist ein Arzneimittel zur Behandlung von Diabetes mellitus bestehend aus einer Formulierung wie oben beschrieben.

Im folgenden wird die Anmeldung anhand einiger Beispiele beschrieben, die keinesfalls beschränkend wirken sollen.

### Figurenlegende:

Fig. 1: Blutzuckersenkende Wirkung von neuen Insulinanaloga gemäß Formel I in Ratten
Fig. 2: Blutzuckersenkende Wirkung von neuen Insulinanaloga gemäß Formel I im Hund
Fig. 3: Blutzuckersenkende Wirkung von YKL205 im Hund
Fig. 4: Zinkabhängigkeit der hypoglykämischen Wirkung von YKL205 im Hund
Fig. 5: Blutzuckersenkende Wirkung von erfindungsgemäßen Insulinanaloga der Formel II in Ratten

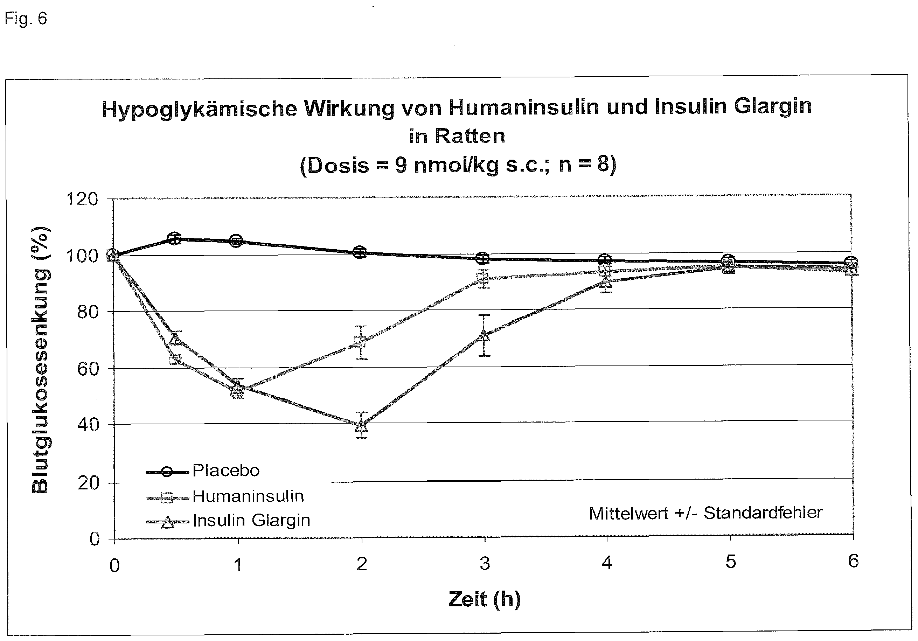

Fig. 6: Blutzuckersenkende Wirkung von Insulin Glargin in Ratten

Beispiele:

Die folgenden Beispiele sollen den Erfindungsgedanken näher erläutern, ohne beschränkend zu wirken.

### Beispiel 1: Studien zur Abfüllung der Lösung unter Verwendung von Stickstoff, Sauerstoff und Abfüllung unter Normalbedingungen

Die Herstellung der Lösung erfolgt indem ca. 25 % 0.1 M HCl vorgelegt werden und 0.2 % Polysorbat 20 Stammlösung dazugegeben wird. Nacheinander werden SAR161271 und die Zinkchlorid Stammlösung hinzugefügt und gerührt. Durch Zugabe von 1 M HCl bei einem pH Wert von pH 2 wird SAR161271 gelöst. Die Lösung wird gerührt, dann erfolgt die Zugabe von 1 M NaOH zur Einstellung des pH Werts auf pH 4.0. Mit Wasser für Injektionszwecke wird auf 90 % der Ansatzgröße aufgefüllt. Zu der Lösung nacheinander Glycerol 85 % und m-Cresol unter Rühren dazugeben. Mit Wasser für Injektionszwecke auf das gewünschte Endgewicht auffüllen. Lösung mittels Spritzenvorsatzfilter filtrieren. Der Ansatz wurde gedrittelt in unbegast (als Referenz), mit Stickstoff begast und mit Sauerstoff begast (als Positivkontrolle). Die Begasung erfolgte als Überlagerung mit dem jeweiligen Gas.

### Unbehandelt

### Gehalt SAR161271

1 M + 5° C: 3.67 mg/ml
1 M + 25° C: 3.46 mg/ml
1 M + 37° C: 3.41 mg/ml

### Verunreinigungen

1 M + 5° C: 3.0 %
1 M + 25° C: 3.6 %
1 M + 37° C: 5.6 %

### Höhermolekulare Proteine

1 M + 5° C: 0.2 %
1 M + 25° C: 0.3 %
1 M + 37° C:1.4 %

### Mit Stickstoff behandelt

### Gehalt SAR161271

1 M + 5° C: 3.73 mg/ml
1 M + 25° C: 3.50 mg/ml
1 M + 37° C: 3.35 mg/ml

### Verunreinigungen

1 M + 5° C: 3.1 %
1 M + 25° C: 3.5 %
1 M + 37° C: 5.2 %

### Höhermolekulare Proteine

1 M + 5° C: 0.2 %
1 M + 25° C: 0.3 %
1 M + 37° C:1.2 %

### Mit Sauerstoff behandelt

### Gehalt SAR161271

1 M + 5° C: 3.54 mg/ml
1 M + 25° C: 3.34 mg/ml
1 M + 37° C: 3.26 mg/ml

### Verunreinigungen

1 M + 5° C: 3.2 %
1 M + 25° C: 3.9 %
1 M + 37° C: 7.2 %

### Höhermolekulare Proteine

1 M + 5° C: 0.2 %
1 M + 25° C: 0.5 %
1 M + 37° C:2.9 %

Die Abfüllung unter Verwendung von Stickstoff zeigte nach 1 Monat keine deutliche Verringerung der Verunreinigungen verglichen mit der unbehandelten Probe. Die Abfüllung unter Verwendung von Sauerstoff zeigte geringfügig höhere Verunreinigungen und höhermolekulare Proteine. Auf Basis dieser Ergebnisse wurde die Abfüllung unter Normalbedingungen gewählt.

### Beispiel 2: Studie zur Stabilität mit 3 verschiedenen Antioxidantien

Die Lösung wurde, wie in Beispiel 1 beschrieben, hergestellt. Zusätzlich zwischen der Zugabe von Glycerol 85 % und m-Cresol die Antioxidantien Methionin oder Glutathion oder Ascorbinsäure zur Formulierung dazugegeben, um den Gehalt des oxidativen Nebenprodukts zu verringern. Die Formulierungen, die entweder Glutathion (0,183 mg/ml) oder Ascorbinsäure (0,105 mg/ml) enthielten zeigten bereits nach 3 Monat Lagerung eine deutliche Verfärbung. Die Methionin (0,089 mg/ml) enthaltende Formulierung zeigte keinerlei Verfärbungen und war nach 1 Monat Lagerung bei 5° C stabil.

### Gehalt SAR161271

1 M + 5° C: 3.43 mg/ml
1 M + 25° C: 3.43 mg/ml
1 M + 37° C: 3.53 mg/ml

### Verunreinigungen

1 M + 5° C: 2.9 %
1 M + 25° C: 3.4 %
1 M + 37° C: 5.7 %

### Höhermolekulare Proteine

1 M + 5° C: 0.2 %
1 M + 25° C: 0.3 %
1 M + 37° C: 1.1 %

### Beispiel 3: Formulierung der amidierten Insulinderivate

Die Beispiele 3 bis 7 dienen nur zur Bestimmung der biologischen, pharmakologischen und physikalisch-chemischen Eigenschaften von Insulinanaloga gemäß Formel I indem zunächst Formulierungen davon bereitgestellt wurden (Beispiel 3) und dann entsprechende Tests vorgenommen wurden (Beispiele 4 bis 7). Es wurde von den Verbindungen wie folgt eine Lösung hergestellt: Das erfindungsgemäße Insulinanalog wurde mit einer Zielkonzentration von 240 ± 5 µM in 1 mM Salzsäure mit 80 µg/mL Zink (als Zinkchlorid) aufgelöst.

Als Lösungsmedium wurden folgende Zusammensetzungen verwendet:
a) 1 mM Salzsäure
b) 1 mM Salzsäure, 5 µg/mL Zink (als Zinkchlorid oder Salzsäure zugesetzt)
c) 1 mM Salzsäure, 10 µg/mL Zink (als Zinkchlorid oder Salzsäure zugesetzt)
d) 1 mM Salzsäure, 15 µg/mL Zink (als Zinkchlorid oder Salzsäure zugesetzt)
e) 1 mM Salzsäure, 30 µg/mL Zink (als Zinkchlorid oder Salzsäure zugesetzt)
f) 1 mM Salzsäure, 80 µg/mL Zink (als Zinkchlorid oder Salzsäure zugesetzt)
g) 1 mM Salzsäure, 120 µg/mL Zink (als Zinkchlorid oder Salzsäure zugesetzt)

Hierzu wurde von dem gefriergetrockneten Material zunächst eine um etwa 30% höhere Menge als aufgrund des Molekulargewichts und der angestrebten Konzentration benötigt eingewogen. Danach wurde die vorliegende Konzentration mittels analytischer HPLC bestimmt und die Lösung anschließend auf das zur Erreichung der Zielkonzentration erforderliche Volumen mit 5 mM Salzsäure mit 80 µg/mL Zink aufgefüllt. Falls erforderlich, wurde dabei der pH-Wert auf 3,5 ± 0,1 nachjustiert. Nach der endgültigen Analyse durch HPLC zur Absicherung der Zielkonzentration von 240 ± 5 µM wurde die fertige Lösung mittels einer Spritze mit einem 0,2 µm Filtervorsatz in ein mit einem Septum und einer Bördelkappe verschlossenes steriles Fläschchen überführt. Für die kurzfristige, einmalige Testung der erfindungsgemäßen Insulinderivate wurde keine Optimierung der Formulierungen, z.B. hinsichtlich eines Zusatzes isotonischer Agentien, Konservierungsmittel oder Puffersubstanzen, vorgenommen.

### Beispiel 4: Evaluierung der blutzuckersenkenden Wirkung von neuen Insulinanaloga in der Ratte

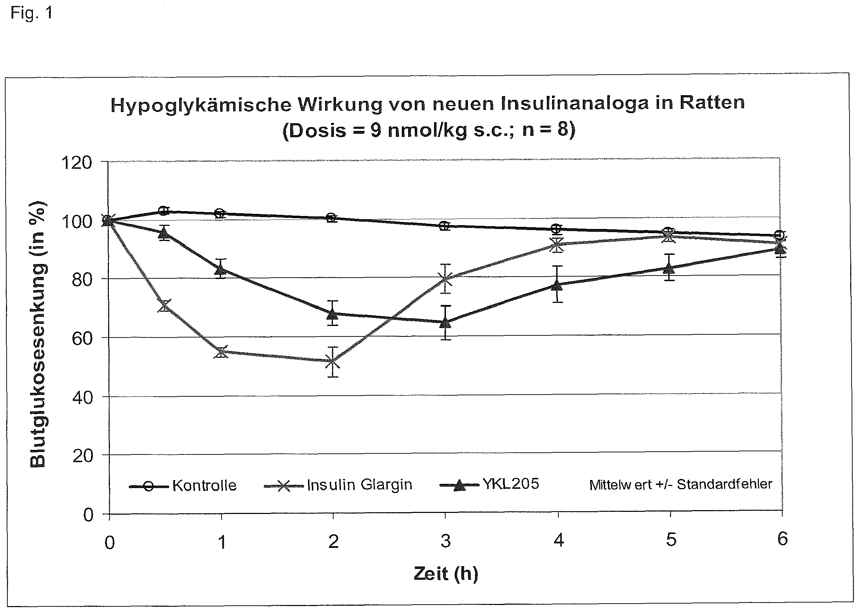

Die blutzuckersenkende Wirkung von ausgewählten neuen Insulinanaloga wird in männlichen, gesunden, normoglykämischen Wistarratten geprüft. Männlichen Ratten wird eine Dosis von 9 nmol/kg eines Insulinanalogons subkutan injiziert. Unmittelbar vor der Injektion des Insulinanalogons und in regelmäßigen Abständen bis zu acht Stunden nach der Injektion werden den Tieren Blutproben entnommen und darin der Blutzuckergehalt bestimmt. Das Experiment zeigt deutlich (vgl. Fig. 1), dass das eingesetzte erfindungsgemäße Insulinanalogon zu einem deutlich verzögerten Wirkeintritt und einer längeren, gleichmäßigen Wirkdauer führt.

### Beispiel 5: Evaluierung der blutzuckersenkenden Wirkung von neuen Insulinanaloga im Hund

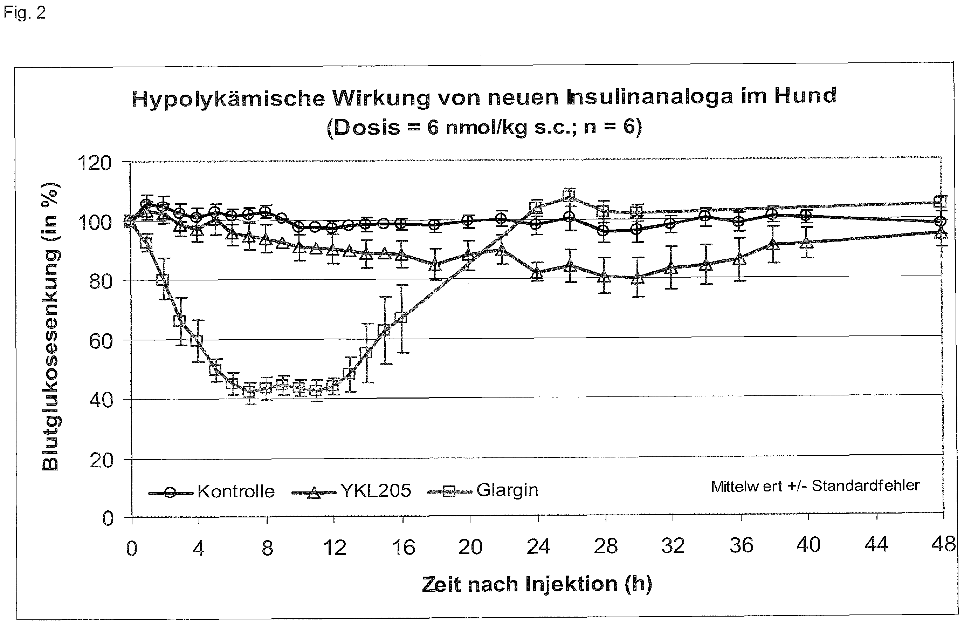

Die blutzuckersenkende Wirkung von ausgewählten neuen Insulinanaloga wird in männlichen, gesunden, normoglykämischen Beaglehunden geprüft. Männlichen Tieren wird eine Dosis von 6 nmol/kg eines Insulinanalogons subkutan injiziert. Unmittelbar vor der Injektion des Insulinanalogons und in regelmäßigen Abständen bis zu achtundvierzig Stunden nach der Injektion werden den Tieren Blutproben entnommen und darin der Blutzuckergehalt bestimmt. Das Experiment zeigt deutlich (vgl. Fig. 2), dass das eingesetzte erfindungsgemäße Insulinanalogon zu einem deutlich verzögerten Wirkeintritt und einer längeren, gleichmäßigen Wirkdauer führt.

### Beispiel 6: Evaluierung der blutzuckersenkenden Wirkung am Hund bei zweifach erhöhter Dosis

Die blutzuckersenkende Wirkung von ausgewählten neuen Insulinanaloga wird in männlichen, gesunden, normoglykämischen Beaglehunden geprüft. Männlichen Tieren wird eine Dosis von 6 nmol/kg und 12nmol/kg eines Insulinanalogons subkutan injiziert. Unmittelbar vor der Injektion des Insulinanalogons und in regelmäßigen Abständen bis zu achtundvierzig Stunden nach der Injektion werden den Tieren Blutproben entnommen und darin der Blutzuckergehalt bestimmt.

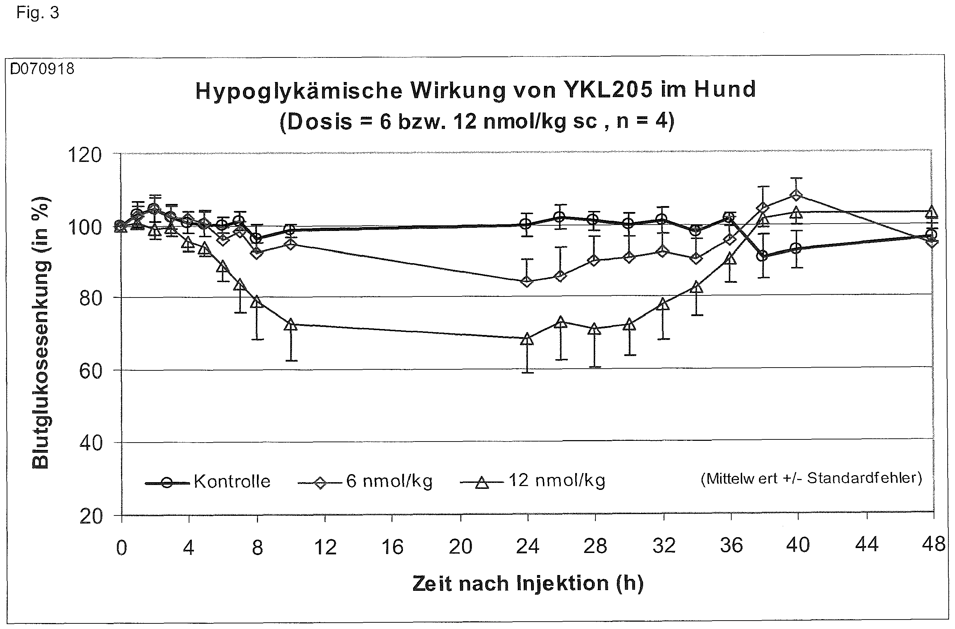

Das Experiment zeigt deutlich (vgl. Fig. 3), dass das eingesetzte erfindungsgemäße Insulinanalogon dosisabhängig wirkt, dass aber trotz zweifach erhöhter Dosis der Wirkungsverlauf flach verläuft, d.h. kein ausgeprägter Tiefpunkt (Nadir) beobachtet wird. Daraus lässt sich ableiten, dass die erfindungsgemäßen Insuline im Vergleich zu bekannten Verzögerungsinsulinen zu deutlich weniger hypoglykämischen Ereignissen führen.

### Beispiel 7: Evaluierung der blutzuckersenkenden Wirkung am Hund bei verschiedenen Zinkkonzentrationen in der Formulierung

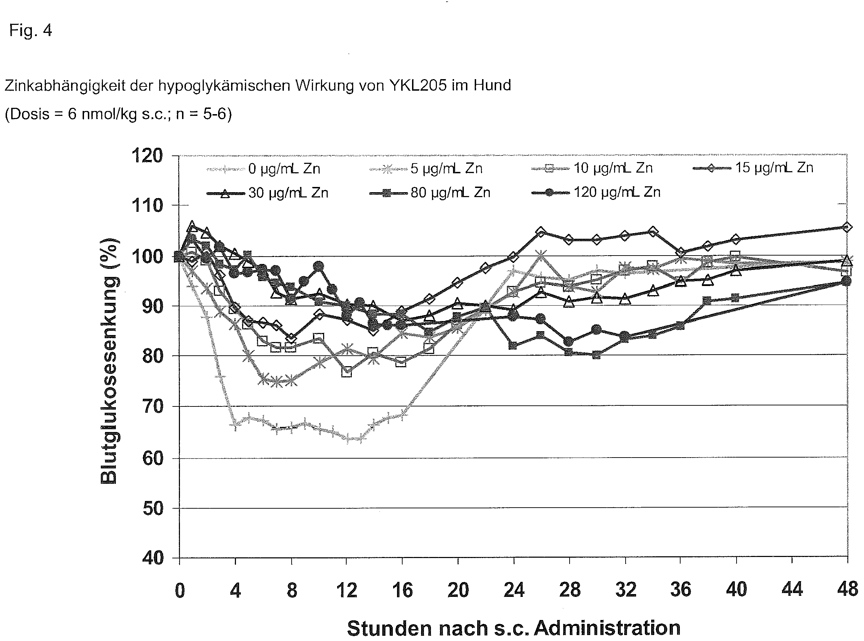

Die Experimente wurden, wie in Beispiel 35 beschrieben, durchgeführt. Figur 4 zeigt das Ergebnis. Danach lässt sich die Zeit - Wirkungskurve des erfindungsgemäßen Insulinanalogons durch den Gehalt an Zinkionen in der Formulierung bei gleicher Insulinkonzentration in der Weise beeinflussen, dass man bei null oder geringem Zinkgehalt einen schnellen Wirkungseintritt beobachtet und die Wirkung über 24 Stunden anhält, während bei höherem Zinkgehalt ein flacher Wirkungseintritt beobachtet wird und die Insulinwirkung deutlich länger als 24 Stunden anhält.

### Beispiel 8: Formulierung der amidierten Insulinderivate

Die Beispiele 8 bis 10 dienen nur zur Bestimmung der biologischen, pharmakologischen und physikalisch-chemischen Eigenschaften von Insulinanaloga gemäß Formel II indem zunächst Formulierungen davon bereitgestellt wurden (Beispiel 8) und dann entsprechende Tests vorgenommen wurden (Beispiele 9 und 10). Das erfindungsgemäße Insulinanalog wurde mit einer Zielkonzentration von 240 ± 5 µM in 1 mM Salzsäure mit 80 µg/mL Zink (als Zinkchlorid) aufgelöst. Hierzu wurde von dem gefriergetrockneten Material zunächst eine um etwa 30% höhere Menge als aufgrund des Molekulargewichts und der angestrebten Konzentration benötigt eingewogen. Danach wurde die vorliegende Konzentration mittels analytischer HPLC bestimmt und die Lösung anschließend auf das zur Erreichung der Zielkonzentration erforderliche Volumen mit 5 mM Salzsäure mit 80 µg/mL Zink aufgefüllt. Falls erforderlich, wurde dabei der pH-Wert auf 3,5 ± 0,1 nachjustiert. Nach der endgültigen Analyse durch HPLC zur Absicherung der Zielkonzentration von 240 ± 5 µM wurde die fertige Lösung mittels einer Spritze mit einem 0,2 µm Filtervorsatz in ein mit einem Septum und einer Bördelkappe verschlossenes steriles Fläschchen überführt. Für die kurzfristige, einmalige Testung der erfindungsgemäßen Insulinderivate wurde keine Optimierung der Formulierungen, z.B. hinsichtlich eines Zusatzes isotonischer Agentien, Konservierungsmittel oder Puffersubstanzen, vorgenommen.

### Beispiel 9: Evaluierung der blutzuckersenkenden Wirkung von neuen Insulinanaloga in der Ratte

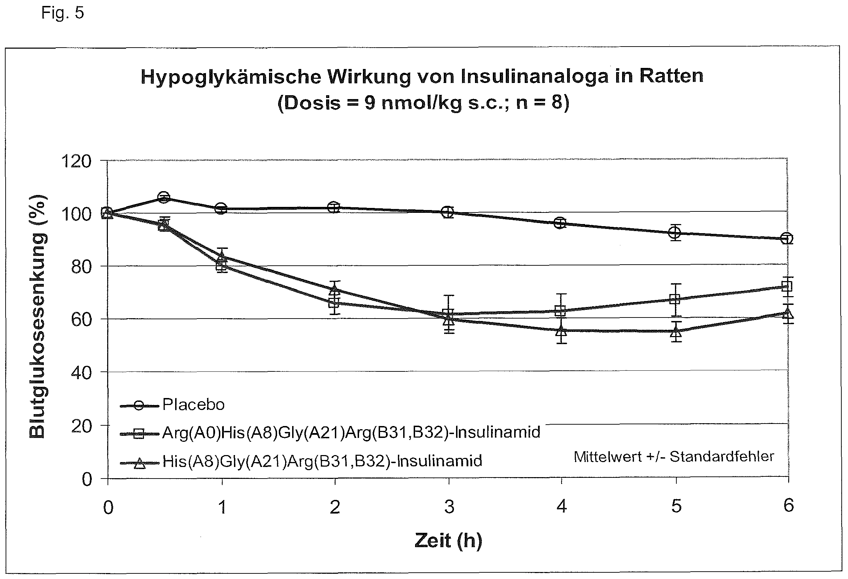

Die blutzuckersenkende Wirkung von ausgewählten neuen Insulinanaloga wird in männlichen, gesunden, normoglykämischen Wistarratten geprüft. Männlichen Ratten wird eine Dosis von 9 nmol/kg eines Insulinanalogons subkutan injiziert. Unmittelbar vor der Injektion des Insulinanalogons und in regelmäßigen Abständen bis zu acht Stunden nach der Injektion werden den Tieren Blutproben entnommen und darin der Blutzuckergehalt bestimmt. Das Experiment zeigt deutlich (vgl. Fig. 5), dass das erfindungsgemäße Insulinanalogon zu einem deutlich verzögerten Wirkeintritt und einer längeren, gleichmäßigen Wirkdauer führt.

### Beispiel 10: Evaluierung der blutzuckersenkenden Wirkung von neuen Insulinanaloga im Hund

Die blutzuckersenkende Wirkung von ausgewählten neuen Insulinanaloga wird in männlichen, gesunden, normoglykämischen Beaglehunden geprüft. Männlichen Tieren wird eine Dosis von 6 nmol/kg eines Insulinanalogons subkutan injiziert. Unmittelbar vor der Injektion des Insulinanalogons und in regelmäßigen Abständen bis zu achtundvierzig Stunden nach der Injektion werden den Tieren Blutproben entnommen und darin der Blutzuckergehalt bestimmt. Das Experiment zeigt deutlich, dass das erfindungsgemäße Insulinanalogon zu einem deutlich verzögerten, flachen Wirkeintritt und einer längeren, gleichmäßigen Wirkdauer führt.

Die folgenden Gegenstände sind Teil der Beschreibung:
1. Wässrige pharmazeutische Formulierung enthaltend ein Insulin, ein Insulinanalogon oder ein Insulinderivat, oder ein pharmakologisch tolerierbares Salz davon, und Methionin.
2. Pharmazeutische Formulierung gemäß Punkt 1, wobei das Insulin ausgewählt wird aus einer Gruppe enthaltend menschliches Insulin, Schweine-Insulin und Insulin des Rindes.
3. Pharmazeutische Formulierung gemäß Punkt 1, wobei das Insulinanalogon ausgewählt wird aus einer Gruppe enthaltend Gly(A21), Arg(B31), Arg(B32)-Humaninsulin, Lys(B3), Glu(B29)-Humaninsulin, Asp(B28)-Humaninsulin, Lys(B28) Pro(B29)-Humaninsulin, Des(B30)-Humaninsulin und ein Insulinanalogon der Formel I 
S ——————— S
1 5 | 10| 15 20
A0 G I V E A5 C C H S I C S L Y A15 L E A18 Y C G
| (SEQ ID NO: 1) A-Kette
S S
|
S S
| |
B-1 B0 B1 B2 B3 B4 H L C G S H L V E A L Y L V C G E R G F F Y
1 5 10 15 20 25

T P B29 B30 B31 B32 (SEQ ID NO: 2)
B-Kette
30
 wobei
   - A0: Lys oder Arg;
   - A5: Asp, Gln oder Glu;
   - A15: Asp, Glu oder Gln;
   - A18: Asp, Glu oder Asn;
   - B-1: Asp, Glu oder eine Aminogruppe;
   - B0: Asp, Glu oder eine chemische Bindung;
   - B1: Asp, Glu oder Phe;
   - B2: Asp, Glu oder Val;
   - B3: Asp, Glu oder Asn;
   - B4: Asp, Glu oder Gln;
   - B29: Lys oder einer chemischen Bindung;
   - B30: Thr oder einer chemischen Bindung;
   - B31: Arg, Lys oder einer chemischen Bindung;
   - B32: Arg-Amid, Lys-Amid oder einer Aminogruppe
   entspricht, wobei zwei Aminosäurereste der Gruppe enthaltend A5, A15, A18, B-1, B0, B1, B2, B3 und B4 gleichzeitig und unabhängig voneinander Asp oder Glu entsprechen.
4. Pharmazeutische Formulierung gemäß Punkt 3, bei der das Insulinanalogon ausgewählt ist aus einer Gruppe enthaltend:
   Arg (A0), His (A8), Glu (A5), Asp (A18), Gly (A21), Arg (B31), Arg (B32)- NH₂ Humaninsulin, Arg (A0), His (A8), Glu (A5), Asp (A18), Gly (A21), Arg (B31), Lys (B32)- NH₂ Humaninsulin, Arg (A0), His (A8), Glu (A15), Asp (A18), Gly (A21), Arg (B31), Arg (B32) - NH₂ Humaninsulin, Arg (A0), His (A8), Glu (A15), Asp (A18), Gly (A21), Arg (B31), Lys (B32) - NH₂ Humaninsulin, Arg (A0), His (A8), Glu(A5), Glu (A15), Gly (A21), Arg (B31), Arg (B32)- NH₂ Humaninsulin, Arg (A0), His (A8), Glu (A5), Glu (A15), Gly (A21), Arg (B31), Lys (B32) - NH₂ Humaninsulin, Arg (A0), His(A8), Glu (A5), Gly (A21), Asp (B3), Arg (B31), Arg (B32) - NH₂ Humaninsulin, Arg (A0), His(A8), Glu (A5), Gly (A21), Asp (B3), Arg (B31), Lys (B32) - NH₂ Humaninsulin, Arg (A0), His (A8), Glu (A15), Gly (A21), Asp (B3), Arg (B31), Arg (B32) - NH₂ Humaninsulin, Arg (A0), His (A8), Glu (A15), Gly (A21), Asp (B3), Arg (B31), Lys (B32) - NH₂ Humaninsulin, Arg (A0), His (A8), Asp (A18), Gly (A21), Asp (B3), Arg (B31), Arg (B32) - NH₂ Humaninsulin, Arg (A0), His (A8), Asp (A18), Gly (A21), Asp (B3), Arg (B31), Lys (B32) - NH₂ Humaninsulin, Arg (A0), His(A8), Gly (A21), Asp (B3), Glu (B4), Arg (B31), Arg (B32) - NH₂ Humaninsulin, Arg (A0), His (A8), Gly (A21), Asp (B3), Glu (B4), Arg (B31), Lys (B32) - NH₂ Humaninsulin, Arg (A0), His (A8), Glu (A5), Gly (A21), Glu (B4), Arg (B31), Arg (B32) - NH₂ Humaninsulin, Arg (A0), His (A8), Glu (A5), Gly (A21), Glu (B4), Arg (B31), Lys (B32) - NH₂ Humaninsulin, Arg (A0), His (A8), Glu (A15), Gly (A21), Glu (B4), Arg (B31), Arg (B32) - NH₂ Humaninsulin, Arg (A0), His (A8), Glu (A15), Gly (A21), Glu (B4), Arg (B31), Lys (B32) - NH₂ Humaninsulin, Arg (A0), His (A8), Asp (A18), Gly (A21), Glu (B4), Arg (B31), Arg (B32) - NH₂ Humaninsulin, Arg (A0), His (A8), Asp (A18), Gly (A21), Glu (B4), Arg (B31), Lys (B32) - NH₂ Humaninsulin, Arg (A0), His (A8), Glu (A5), Gly (A21), Glu (B0), Arg (B31), Arg (B32) - NH₂ Humaninsulin, Arg (A0), His (A8), Glu (A5), Gly (A21), Glu (B0), Arg (B31), Lys (B32)- NH₂ Humaninsulin, Arg (A0), His (A8), Glu (A15), Gly (A21), Glu (B0), Arg (B31), Arg (B32) - NH₂ Humaninsulin, Arg (A0), His (A8), Glu (A15), Gly (A21), Glu (B0), Arg (B31), Lys (B32) - NH₂ Humaninsulin, Arg (A0), His (A8), Asp (A18), Gly (A21), Glu (B0), Arg (B31), Arg (B32) - NH₂ Humaninsulin, Arg (A0), His (A8), Asp (A18) ,Gly (A21), Glu (B0), Arg (B31), Lys (B32) - NH₂ Humaninsulin, Arg (A0), His (A8), Glu (A5), Gly (A21), Asp (B1), Arg (B31), Arg (B32) - NH₂ Humaninsulin, Arg (A0), His (A8), Glu (A5), Gly (A21), Asp (B1), Arg (B31), Lys (B32) - NH₂ Humaninsulin, Arg (A0), His (A8), Glu (A15), Gly (A21), Asp (B1), Arg (B31), Arg(B32) - NH₂ Humaninsulin, Arg (A0), His (A8), Glu (A15), Gly (A21), Asp (B1), Arg (B31), Lys (B32) - NH₂ Humaninsulin, Arg (A0), His (A8), Asp (A18), Gly (A21), Asp (B1), Arg (B31), Arg (B32) - NH₂ Humaninsulin, Arg (A0), His (A8), Asp (A18), Gly (A21), Asp (B1), Arg (B31), Lys (B32) - NH₂ Humaninsulin, Arg (A0), His (A8), Gly (A21), Glu (B0), Asp (B1), Arg (B31), Arg (B32) - NH₂ Humaninsulin, Arg (A0), His (A8), Gly (A21), Glu (B0), Asp (B1), Arg (B31), Lys (B32) - NH₂ Humaninsulin, Arg (A0), His (A8), Asp (A18), Gly (A21), Asp (B3), Arg (B30), Arg (B31) - NH₂ Humaninsulin, Arg (A0), His (A8), Asp (A18), Gly (A21), Asp (B3), Arg (B30), Lys (B31) - NH₂ Humaninsulin.
5. Pharmazeutische Formulierung gemäß Punkt 1, wobei das Insulinanalogon ausgewählt wird aus einer Gruppe enthaltend ein Insuiinanalogon der Formel II 
S S
1 5 10 15 20
A-1 A0 A1 I V E A5 C C H S I C S L Y A15 L E A18 Y C A21
A-Kette
(SEQ ID NO: 3)
S S
S S
B-1 B0 B1 V B3 B4 H L C G S H L V E A L Y L V C G E R G F F Y
1 5 10 15 20 25
T P B29 B30 B31 B32 (SEQ ID NO: 4) B-Kette
30
 wobei
   A-1 Lys, Arg oder einer Aminogruppe;
   A0 Lys, Arg oder einer chemischen Bindung;
   A1 Arg oder Gly;
   A5 Asp, Glu oder Gln;
   A15 Asp, Glu oder Gln;
   A18 Asp, Glu oder Asn;
   A21 Ala, Ser, Thr oder Gly;
   B-1 Asp, Glu oder eine Aminogruppe;
   B0 Asp, Glu oder eine chemische Bindung;
   B1 Asp, Glu, Phe oder eine chemische Bindung;
   B3 Asp, Glu oder Asn;
   B4 Asp, Glu oder Gln;
   B29 Arg, Lys oder einer Aminosäure ausgewählt aus einer Gruppe enthaltend die Aminosäuren Phe, Ala, Thr, Ser, Val, Leu, Glu oder Asp, oder einer chemischen Bindung;
   B30 Thr oder einer chemischen Bindung;
   B31 Arg, Lys oder einer chemischen Bindung;
   B32 Arg-Amid oder Lys-Amid
   entspricht, wobei nicht mehr als ein Aminosäurerest der Gruppe enthaltend A5, A15, A18, B-1, B0, B1, B2, B3 und B4 gleichzeitig und unabhängig voneinander Asp oder Glu entsprechen.
6. Pharmazeutische Formulierung gemäß Punkt 5, bei der das Insulinanalogon ausgewählt ist aus einer Gruppe enthaltend:
   Arg (A-1), Arg (A0), Glu (A5), His (A8), Gly (A21), Arg (B30) - NH₂ Humaninsulin, Arg (A-1), Arg (A0), Glu (A5), His (A8), Gly (A21), Lys (B30) - NH₂ Humaninsulin, Arg (A-1), Arg (A0), Glu (A15), His (A8), Gly (A21), Arg (B30) - NH₂ Humaninsulin, Arg (A-1), Arg (A0), Glu (A15), His (A8), Gly (A21), Lys (B30) - NH₂ Humaninsulin, Arg (A-1), Arg (A0), Asp (A18), His (A8), Gly (A21), Arg (B30) - NH₂ Humaninsulin, Arg (A-1), Arg (A0), Asp (A18), His (A8), Gly (A21), Arg (B30) - NH₂ Humaninsulin, Arg (A-1), Arg (A0), His (A8), Gly (A21), Glu (B0), Arg (B30) - NH₂ Humaninsulin, Arg (A-1), Arg (A0), His (A8), Gly (A21), Glu (B0), Lys (B30) - NH₂ Humaninsulin, Arg (A-1), Arg (A0), His (A8), Gly (A21), Asp (B3), Arg (B30) - NH₂ Humaninsulin, Arg (A-1), Arg (A0), His (A8), Gly (A21), Asp (B3), Lys (B30) - NH₂ Humaninsulin, Arg (A-1), Arg (A0), His (A8), Gly (A21), Glu (B4), Arg (B30) - NH₂ Humaninsulin, Arg (A-1), Arg (A0), His (A8), Gly (A21), Glu (B4), Lys (B30) - NH₂ Humaninsulin, Arg (A0), His (A8), Gly (A21), Arg (B31), Arg (B32) - NH₂ - Humaninsulin, Arg (A0), His (A8), Gly (A21), Arg (B31), Lys (B32) - NH₂ - Humaninsulin, Arg (A0), Glu (A5), His (A8), Gly (A21), Arg (B31), Arg (B32) - NH₂ - Humaninsulin, Arg (A0), Glu (A5), His (A8), Gly (A21), Arg (B31), Lys (B32) - NH₂ - Humaninsulin, Arg (A0), Asp (A18), His (A8), Gly (A21), Arg (B31), Arg (B32) - NH₂ - Humaninsulin, Arg (A0), Asp (A18), His (A8), Gly (A21), Arg (B31), Lys (B32) - NH₂ - Humaninsulin, Arg (A0), Glu (A15), His (A8), Gly (A21), Arg (B31), Arg (B32) - NH₂ - Humaninsulin, Arg (A0), Glu (A15), His (A8), Gly (A21), Arg (B31), Lys (B32) - NH₂ - Humaninsulin, Arg (A0), His (A8), Gly (A21), Asp (B3), Arg (B31), Arg (B32) - NH₂ - Humaninsulin, Arg (A0), His (A8), Gly (A21), Asp (B3), Arg (B31), Lys (B32) - NH₂ - Humaninsulin, Arg (A0), His (A8), Gly (A21), Glu (B4), Arg (B31), Arg (B32) - NH₂ - Humaninsulin, Arg (A0), His (A8), Gly (A21), Glu (B4), Arg (B31), Lys (B32) - NH₂ - Humaninsulin, Arg (A0), His (A8), Gly (A21), Glu (B0), Arg (B31), Arg (B32) - NH₂ - Humaninsulin, Arg (A0), His (A8), Gly (A21), Glu (B0), Arg (B31), Lys (B32) - NH₂ - Humaninsulin, Arg (A0), His (A8), Gly (A21), Arg (B30) - NH₂ - Humaninsulin, Arg (A0), His (A8), Gly (A21), Lys (B30) - NH₂ - Humaninsulin, Arg (A-1), Arg (A0), His (A8), Gly (A21), Arg (B30) - NH₂ - Humaninsulin, Arg (A-1), Arg (A0), His (A8), Gly (A21), Lys (B30) - NH₂ - Humaninsulin, Arg (A0), Arg (A1), His (A8), Gly (A21), Arg (B30) - NH₂ - Humaninsulin, Arg (A0), Arg (A1), His (A8), Gly (A21), Lys (B30) - NH₂ - Humaninsulin, His (A8), Gly (A21), Arg (B31), Arg (B32) - NH₂ - Humaninsulin.
7. Pharmazeutische Formulierung gemäß Punkt 1, wobei das Insulinderivat ausgewählt wird aus einer Gruppe enthaltend B29-N-myristoyl-des(B30) Humaninsulin, B29-N-palmitoyl-des(B30) Humaninsulin, B29-N-myristoyl Humaninsulin, B29-N-palmitoyl Humaninsulin, B28-N-myristoyl Lys^{B28}Pro^{B29} Humaninsulin, B28-N-palmitoyl-Lys^{B28}Pro^{B29} Humaninsulin, B30-N-myristoyl-Thr^{B29}Lys^{B30} Humaninsulin, B30-N-palmitoyl- Thr^{B29}Lys^{B30} Humaninsulin, B29-N-(N-palmitoyl-γ-glutamyl)-des(B39) Humaninsulin, B29-N-(N-lithocholyl-γ-glutamyl)-des(B30) Humaninsulin, B29-N-(ω-carboxyheptadecanoyl)-des(B30) Humaninsulin and B29-N-(ω-carboxyheptadecanoyl) Humaninsulin.
8. Pharmazeutische Formulierung gemäß einem oder mehreren der Punkte 1 bis 7, die
   0,001 bis 0,2 mg/ml Zink,
   0,1 bis 5,0 mg/ml eines Konservierungsmittels und
   5,0 bis 100 mg/ml eines Isotonisierungsmittels enthält und
   deren pH-Wert 5 oder kleiner ist.
9. Pharmazeutische Formulierung gemäß einem oder mehreren der Punkte 1 bis 8 in dem ein Konservierungsmittel ausgewählt aus einer Gruppe enthaltend Phenol, m-Cresol, Chlorkresol, Benzylalkohol, Parabene vorhanden ist.
10. Pharmazeutische Formulierung gemäß einem der Punkte 1 bis 9, in dem ein Isotonisierungsmittel ausgewählt aus einer Gruppe enthaltend Mannitol, Sorbitol, Lactose, Dextrose, Trehalose, Natriumchlorid, Glycerol vorhanden ist.
11. Pharmazeutische Formulierung gemäß einem der Punkte 1 bis 10, mit einem pH-Wert im Bereich von pH 2,5 - 4,5.
12. Pharmazeutische Formulierung gemäß einem der Punkte 1 bis 11, mit einem pH-Wert im Bereich von pH 3,0 - 4,0.
13. Pharmazeutische Formulierung gemäß einem der Punkte 1 bis 12, mit einem pH-Wert im Bereich von pH 3,75.
14. Pharmazeutische Formulierung gemäß einem der Punkte 1 bis 13, wobei das Insulin, das Insulinanalogon und/oder das Insulinderivat in einer Konzentration von 240 - 3000 nmol/ml vorliegt
15. Pharmazeutische Formulierung gemäß einem oder mehreren der Punkte 1 bis 14, bei der Glycerol in einer Konzentration von 20 bis 30 mg/ml vorliegt.
16. Pharmazeutische Formulierung gemäß einem oder mehreren der Punkte 1 bis 15, bei der Glycerol in einer Konzentration von 25 mg/ml vorliegt.
17. Pharmazeutische Formulierung gemäß einem oder mehreren der Punkte 1 bis 16, bei der m-Cresol in einer Konzentration von 1 bis 3 mg/ml vorliegt.
18. Pharmazeutische Formulierung gemäß einem oder mehreren der Punkte 1 bis 17, bei der m-Cresol in einer Konzentration von 2 mg/ml vorliegt.
19. Pharmazeutische Formulierung gemäß einem oder mehreren der Punkte 1 bis 18, bei der Zink in einer Konzentration 0,01 oder 0,03 oder 0,08 mg/ml vorliegt.
20. Pharmazeutische Formulierung nach einem oder mehreren der Punkte 1 bis 19, bei der noch zusätzlich ein Glucagon-Like Peptide-1 (GLP1) oder ein Analogon oder Derivat davon, oder Exendin-3 bzw. -4 oder ein Analogon oder Derivat davon enthalten ist.
21. Pharmazeutische Formulierung nach Punkt 20, bei dem zusätzlich Exendin-4 enthalten ist.
22. Pharmazeutische Formulierung gemäß Punkt 20, bei dem ein Analogon von Exendin-4 ausgewählt wird aus einer Gruppe enthaltend
   H-desPro³⁶-Exend in-4-Lys₆-NH₂,
   H-des(Pro^{36,37})-Exendin-4-Lys₄-NH₂ und
   H-des(Pro^{36,37})-Exendin-4-Lys₅-NH₂,
   oder ein pharmakologisch tolerierbares Salz davon.
23. Pharmazeutische Formulierung gemäß Punkt 20, bei dem ein Analogon von Exendin-4 ausgewählt wird aus einer Gruppe enthaltend
   desPro³⁶[Asp²⁸]Exendin-4 (1-39),
   desPro³⁶ [IsoAsp²⁸]Exendin-4 (1-39),
   desPro³⁶ [Met(O)¹⁴, Asp²⁸]Exendin-4 (1-39),
   desPro³⁶ [Met(O)¹⁴, IsoAsp²⁸]Exendin-4 (1-39),
   desPro³⁶ [Trp(O₂)²⁵, Asp²⁸]Exendin-2 (1-39),
   desPro³⁶ [Trp(O₂)²⁵, IsoAsp²⁸]Exendin-2 (1-39),
   desPro³⁶ [Met(O)¹⁴Trp(O₂)²⁵ , Asp²⁸]Exendin-4 (1-39) und
   desPro³⁶ [Met(O)14Trp(O₂)²⁵, IsoAsp²⁸]Exendin-4 (1-39),
   oder ein pharmakologisch tolerierbares Salz davon.
24. Pharmazeutische Formulierung gemäß Punkt 23, bei denen an die C-Termini der Analoga von Exendin-4 das Peptid -Lys₆-NH₂ angefügt ist.
25. Pharmazeutische Formulierung gemäß Punkt 20, bei dem ein Analogon von Exendin-4 ausgewählt wird aus einer Gruppe enthaltend
   H-(Lys)₆- des Pro³⁶ [Asp²⁸]Exendin-4(1-39)-Lys₆-NH₂
   des Asp²⁸Pro³⁶, Pro³⁷, Pro³⁸ Exendin-4(1-39) -NH₂,
   H-(Lys)₆- des Pro³⁶, Pro³⁷, Pro³⁸ [Asp²⁸]Exendin-4(1-39) -NH₂,
   H-Asn-(Glu)₅ des Pro³⁶, Pro³⁷, Pro³⁸ [Asp²⁸]Exendin-4(1-39) -NH₂,
   des Pro³⁶, Pro³⁷, Pro³⁸ [Asp²⁸]Exendin-4(1-39)-(Lys)₆-NH₂,
   H-(Lys)₆- des Pro³⁶, Pro³⁷, Pro³⁸ [Asp²⁸]Exendin-4(1-39)-(Lys)₆-NH₂,
   H-Asn-(Glu)₅- des Pro³⁶, Pro³⁷, Pro³⁸ [Asp²⁸]Exendin-4(1-39)-(Lys)₆-NH₂,
   H-(Lys)₆- des Pro³⁶ [Trp(O₂)²⁵, Asp²⁸]Exendin-4(1-39)-Lys₆-NH₂,
   H- des Asp²⁸ Pro³⁶, Pro³⁷, Pro³⁸ [Trp(O₂)²⁵]Exendin-4(1-39) -NH₂,
   H-(Lys)₆- des Pro³⁶, Pro³⁷, Pro³⁸ [Trp(O₂)²⁵, Asp²⁸]Exendin-4(1-39) -NH₂,
   H-Asn-(Glu)₅- des Pro³⁶, Pro³⁷, Pro³⁸ [Trp(O₂)²⁵, Asp²⁸]Exendin-4(1-39) -NH₂,
   des Pro³⁶, Pro³⁷, Pro³⁸ [Trp(O₂)²⁵, Asp²⁸]Exendin-4(1-39)-(Lys)₆-NH₂,
   H-(Lys)₆- des Pro³⁶, Pro³⁷, Pro³⁸ [Trp(O₂)²⁵, Asp²⁸]Exendin-4(1-39)-(Lys)₆-NH₂,
   H-Asn-(Glu)₅- des Pro³⁶, Pro³⁷, Pro³⁸ [Trp(O₂)²⁵, Asp²⁸]Exendin-4(1-39)-(Lys)₆-NH₂,
   H-(Lys)₆- des Pro³⁶ [Met(O)¹⁴, Asp²⁸]Exendin-4(1-39)-Lys₆-NH₂,
   des Met(O)¹⁴ Asp²⁸ Pro³⁶, Pro³⁷, Pro³⁸ Exendin-4(1-39) -NH₂,
   H-(Lys)₆- des Pro³⁶, Pro³⁷, Pro³⁸ [Met(O)¹⁴, Asp²⁸]Exendin-4(1-39) -NH₂,
   H-Asn-(Glu)₅- des Pro³⁶, Pro³⁷, Pro³⁸ [Met(O)¹⁴, Asp²⁸] Exendin-4(1-39) -NH₂,
   des Pro³⁶, Pro³⁷, Pro³⁸ [Met(O)¹⁴, Asp²⁸]Exendin-4(1-39)-(Lys)₆-NH₂,
   H-(Lys)⁶- des Pro³⁶, Pro³⁷, Pro³⁸ [Met(O)¹⁴, Asp²⁸]Exendin-4(1-39)-Lys₆-NH₂,
   H-Asn-(Glu)₅ des Pro³⁶, Pro³⁷, Pro³⁸ [Met(O)¹⁴, Asp²⁸] Exendin-4(1-39)-(Lys)₆-NH₂,
   H-(Lys)₆- des Pro³⁶ [Met(O)¹⁴, Trp(O₂)²⁵, Asp²⁸]Exendin-4(1-39)-Lys₆-NH₂,
   des Asp²⁸ Pro³⁶, Pro³⁷, Pro³⁸ [Met(O)¹⁴, Trp(O₂)²⁵]Exendin-4(1-39) -NH₂,
   H-(Lys)₆- des Pro³⁶_{'} Pro³⁷, Pro³⁸ [Met(O)¹⁴, Trp(O₂)²⁵, Asp²⁸]Exendin-4(1-39) -NH₂,
   H-Asn-(Glu)₅- des Pro³⁶, Pro³⁷, Pro³⁸ [Met(O)¹⁴, Asp²⁸ Exendin-4(1-39) -NH₂,
   des Pro³⁶, Pro³⁷, Pro³⁸ [Met(O)¹⁴, Trp(O₂)²⁵, Asp²⁸]Exendin-4(1-39)-(Lys)₆-NH₂,
   H-(Lys)₆- des Pro³⁶_{'} Pro³⁷, Pro³⁸ [Met(O)¹⁴, Trp(O₂)²⁵, Asp²⁵]Exendin-4(1-39)-(Lys)₆-NH₂,
   H-Asn-(Glu)₅- des Pro³⁶, Pro³⁷, Pro³⁸ [Met(O)¹⁴, Trp(O₂)²⁵, Asp²⁸ Exendin-4(1-39)-(Lys)₆-NH₂,
   oder ein pharmakologisch tolerierbares Salz davon.
26. Pharmazeutische Formulierung nach Punkt 20, bei dem zusätzlich Arg³⁴, Lys²⁶ (N^{ε}(γ-glutamyl(N^{α}-hexadecanoyl))) GLP-1 (7-37) [liraglutidel oder ein pharmakologisch tolerierbares Salz davon enthalten ist.
27. Pharmazeutische Formulierung nach einem oder mehreren der Punkte 1 bis 24, bei der Methionin im Konzentrationsbereich von bis zu 10 mg/ml vorhanden ist.
28. Pharmazeutische Formulierung nach Punkt 25, bei der Methionin im Konzentrationsbereich von bis zu 3 mg/ml vorhanden ist.
29. Verfahren zur Herstellung einer Formulierung gemäß einem oder mehreren der Punkte 1 bis 26, bei dem
   (a) die Komponenten in eine wässrige Lösung eingebracht werden und
   (b) der pH-Wert eingestellt wird.
30. Verwendung einer Formulierung gemäß einem oder mehreren der Punkte 1 bis 26 zur Behandlung von Diabetes mellitus.
31. Arzneimittel zur Behandlung von Diabetes mellitus bestehend aus einer Formulierung gemäß einem oder mehreren der Punkte 1 bis 26.

## Patentansprüche

1. Wässrige pharmazeutische Formulierung enthaltend ein Insulinanalogon oder ein pharmakologisch tolerierbares Salz davon, und Methionin, wobei das Insulinanalogon ausgewählt ist aus einer Gruppe bestehend aus Gly(A21), Arg(B31), Arg(B32)-Humaninsulin, Lys(B3), Glu(B29)-Humaninsulin, Asp(B28)-Humaninsulin, Lys(B28) Pro(B29)-Humaninsulin, Des(B30)-Humaninsulin und einem Insulinanalogon der Formel I 
S ——————— S
1 5 | 10| 15 20
A0 G I V E A5 C C H S I C S L Y A15 L E A18 Y C G
| (SEQ ID NO: 1) A-Kette
S S
|
S S
| |
B-1 B0 B1 B2 B3 B4 H L C G S H L V E A L Y L V C G E R G F F Y
1 5 10 15 20 25

T P B29 B30 B31 B32 (SEQ ID NO: 2)
B-Kette
30
 wobei
A0 Lys oder Arg;
A5 Asp, Gln oder Glu;
A15 Asp, Glu oder Gln;
A18 Asp, Glu oder Asn;
B-1 Asp, Glu oder einer Aminogruppe;
B0 Asp, Glu oder einer chemischen Bindung;
B1 Asp, Glu oder Phe;
B2 Asp, Glu oder Val;
B3 Asp, Glu oder Asn;
B4 Asp, Glu oder Gin;
B29 Lys oder einer chemischen Bindung;
B30 Thr oder einer chemischen Bindung;
B31 Arg, Lys oder einer chemischen Bindung;
B32 Arg-Amid, Lys-Amid oder einer Aminogruppe
entspricht, wobei zwei Aminosäurereste der Gruppe bestehend aus A5, A15, A18, B1, B0, B1, B2, B3 und B4 gleichzeitig und unabhängig voneinander Asp oder Glu entsprechen.

2. Pharmazeutische Formulierung gemäß Anspruch 1, die
0,001 bis 0,2 mg/ml Zink,
0,1 bis 5,0 mg/ml eines Konservierungsmittels und
5,0 bis 100 mg/ml eines Isotonisierungsmittels enthält.

3. Pharmazeutische Formulierung gemäß Anspruch 1 oder 2, in der ein Konservierungsmittel ausgewählt aus einer Gruppe bestehend aus Phenol, m-Cresol, Chlorcresol, Benzylalkohol, Parabenen vorhanden ist.

4. Pharmazeutische Formulierung gemäß einem der Ansprüche 1 bis 3, in der ein Isotonisierungsmittel ausgewählt aus einer Gruppe bestehend aus Mannitol, Sorbitol, Lactose, Dextrose, Trehalose, Natriumchlorid, Glycerol vorhanden ist.

5. Pharmazeutische Formulierung gemäß einem der Ansprüche 1 bis 4, wobei das Insulinanalogon in einer Konzentration von 240 - 3000 nmol/ml vorliegt.

6. Pharmazeutische Formulierung gemäß einem der Ansprüche 1 bis 5, bei der Glycerol in einer Konzentration von 20 bis 30 mg/ml, vorzugsweise in einer Konzentration von 25 mg/ml vorliegt.

7. Pharmazeutische Formulierung gemäß einem der Ansprüche 1 bis 6, bei der m-Cresol in einer Konzentration von 1 bis 3 mg/ml, vorzugsweise in einer Konzentration von 2 mg/ml vorliegt.

8. Pharmazeutische Formulierung gemäß einem der Ansprüche 1 bis 7, bei der Zink in einer Konzentration 0,01 oder 0,03 oder 0,08 mg/ml vorliegt.

9. Pharmazeutische Formulierung gemäß einem der Ansprüche 1 bis 8, bei der Methionin im Konzentrationsbereich von bis zu 10 mg/ml, vorzugsweise von bis zu 3 mg/ml vorhanden ist.

10. Pharmazeutische Formulierung gemäß einem der Ansprüche 1 bis 9, wobei das Insulinanalogon Lys(B3), Glu(B29)-Humaninsulin ist.

11. Pharmazeutische Formulierung gemäß einem der Ansprüche 1 bis 9, wobei das Insulinanalogon Lys(B28) Pro(B29)-Humaninsulin ist.

12. Verfahren zur Herstellung einer Formulierung gemäß einem der Ansprüche 1 bis 11, bei dem
(a) die Komponenten in eine wässrige Lösung eingebracht werden und
(b) der pH-Wert eingestellt wird.

13. Verwendung einer Formulierung gemäß einem der Ansprüche 1 bis 11 zur Herstellung eines Arzneimittels zur Behandlung von Diabetes mellitus.

14. Arzneimittel zur Anwendung in der Behandlung von Diabetes mellitus bestehend aus einer Formulierung gemäß einem der Ansprüche 1 bis 11.
